(19) **Europäisches Patentamt**

**European Patent Office**

**Office européen des brevets**

(11) **EP 1 034 776 A1**

(12) **DEMANDE DE BREVET EUROPEEN**

(43) Date de publication:
**13.09.2000 Bulletin 2000/37**

(51) Int Cl.⁷: **A61K 7/06**

(21) Numéro de dépôt: **00400198.8**

(22) Date de dépôt: **26.01.2000**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **02.03.1999 FR 9902564**

(71) Demandeur: **L'OREAL**
**75008 Paris (FR)**

(72) Inventeur: **Tournilhac, Florence**
**75011 Paris (FR)**

(74) Mandataire: **Lhoste, Catherine**
**L'OREAL-DPI**
**6 rue Bertrand Sincholle**
**92585 Clichy Cédex (FR)**

(54) **Utilisation dans une composition pour la peau d'un copolymère d'oléfines à cristallisation controlée**

(57) L'invention se rapporte à l'utilisation dans une composition à appliquer sur la peau, qui comprend une phase grasse liquide d'une quantité efficace d'un copolymère d'oléfines cristallin, ayant un taux de cristallinité inférieur ou égal à 50%, pour limiter, voir supprimer, la migration de la composition. Cette composition est plus spécialement destinée au maquillage de la peau et des lèvres et se présente notamment sous forme d'un fond de teint, un produit anti-cerne, un rouge à lèvres ou un fard à paupières, coulés en stick ou en coupelle.

EP 1 034 776 A1

**Description**

La présente invention a trait à l'utilisation d'un copolymère d'oléfine dans une composition destinée en particulier au domaine cosmétique. Plus spécialement, cette composition est destinée au soin et/ou au maquillage de la peau aussi bien du visage que du corps humain et des lèvres.

Cette composition peut se présenter notamment sous forme de produit coulé en stick ou en coupelle comme les rouges ou baumes à lèvres, les fonds de teint coulés, les produits anti-cernes, les fards à paupières ou à joues, sous forme de pâte ou de crème plus ou moins fluide comme les fonds de teint ou rouges à lèvres fluides, les eyesliners, les produits de maquillage du corps (tatouage semi-pemanent par exemple), les compositions de protection solaire ou de coloration de la peau, ou de poudres libres ou compactées.

Les produits de maquillage ou de soin de la peau ou des lèvres des êtres humains comme les fonds de teint ou les rouges à lèvres, contiennent généralement des phases grasses telles que des cires et des huiles, des pigments et/ou charges et, éventuellement des additifs comme des actifs cosmétiques ou dermatologiques. Elles peuvent aussi contenir des produits dits "pâteux", de consistance souple, permettant d'obtenir des pâtes, colorées ou non.

L'emploi de cires présente certains inconvénients. En particulier, le taux de cristallinité de ces cires est difficilement contrôlable et les cristallites présents sont de grandes dimensions. Par suite, l'emploi de telles cires dans les compositions pour la peau, en particulier cosmétiques, entraîne une matification de ces compositions et par conséquent du film appliqué sur la peau ou les lèvres.

Pour remédier à ce problème, on a proposé l'emploi de polyoléfines classiques à la place des cires. Mais là encore, le taux de cristallinité est trop élevé et difficilement contrôlable. En outre, la taille et la morphologie des cristallites, majoritairement de type sphérulite, nuisent à l'obtention de compositions ayant les propriétés cosmétiques souhaitées.

De plus, ces compositions de maquillage, lorsqu'elles sont appliquées sur la peau ou les lèvres, et en particulier les matières colorantes comme les pigments et les huiles de ces compositions ont tendance à migrer, c'est-à-dire à se propager à l'intérieur des rides et des ridules de la peau qui entourent les lèvres et les yeux, entraînant un effet inesthétique. L'apparition de ces traces a tendance à écarter certaines femmes de l'utilisation de ce type de maquillage.

Il subsiste donc le besoin d'une composition ne présentant pas les inconvénients ci-dessus, et en particulier ne migrant pas, présentant un aspect plus ou moins brillant, adapté au désir de la consommatrice, ne desséchant pas au cours du temps la peau ou les lèvres sur lesquelles elle est appliquée et ne provoquant pas d'inconfort.

La demanderesse a constaté, de façon tout à fait surprenante, que l'utilisation d'au moins un copolymère d'oléfines à cristallisation contrôlée et modérée, soluble ou dispersable dans une phase grasse, dans une composition pour la peau ou les lèvres permettait d'obtenir un film, de très bonne tenue, ne migrant peu ou pas du tout, résistant à l'eau, tout en étant très agréable à l'application et à porter tout au long de la journée. Le film est notamment souple, flexible et non collant.

Les copolymères à cristallisation contrôlée et modérée, selon l'invention, présentent une taille et une morphologie spécifiques des cristallites. Ils comportent peu ou pas de cristallites de type sphérulite de grandes dimensions, mais au contraire principalement des cristallites de type lamellaire ou micelle frangée.

La présente invention a donc pour objet l'utilisation dans une composition pour matières kératiniques, comprenant une phase grasse liquide, d'une quantité efficace d'au moins un copolymère soluble ou dispersable dans la phase grasse liquide, choisi parmi les copolymères d'oléfines cristallins ayant un taux de cristallinité au plus égal à 50 %, de préférence allant de 5 à 40%, et mieux allant de 10 à 35 % pour limiter et/ou supprimer la migration de ladite composition.

L'invention a aussi pour objet l'utilisation dans une composition non migrante pour matières kératiniques, comprenant une phase grasse liquide d'une quantité efficace d'au moins un copolymère soluble ou dispersable dans la phase grasse liquide, choisi parmi les copolymères d'oléfines cristallins ayant un taux de cristallinité au plus égal à 50 %, de préférence allant de 5 à 40%, et mieux allant de 10 à 35%.

Par « phase grasse liquide » on entend tout milieu non aqueux liquide à température ambiante (20-25°C) et pression atmosphérique, et en particulier les huiles et les solvants.

Cette composition est en particulier une composition physiologiquement acceptable et plus spécialement une composition cosmétique. Elle contient donc des ingrédients compatibles avec la peau, y compris celle du cuir chevelu et des lèvres, et les fibres kératiniques ou phanères.

De préférence, cette composition contient, en outre, au moins une matière colorante.

Le ou les copolymères d'oléfines sont présents en une quantité efficace ou suffisante pour obtenir notamment un film de bonne tenue et/ou ne migrant pas dans les plis de la peau comme les rides, ridules situées autour des lèvres et des yeux (paupières notamment).

Le ou les copolymères d'oléfines cristallins utilisés dans la composition de la présente demande peuvent être tout copolymère d'oléfines, à savoir un copolymère comportant uniquement des motifs oléfiniques, ayant un caractère

2

cristallin contrôlé et modéré, c'est-à-dire un taux de cristallinité au plus égal à 50%, de préférence allant de 5 à 40%, et mieux allant de 10 à 35%. Ces copolymères gonflés par une ou plusieurs huiles « solvatantes » se comportent comme un élastomère que l'on peut introduire aisément dans une composition de l'art antérieur en vue d'en diminuer sa migration.

Ces copolymères sont généralement des élastomères ou des plastomères et peuvent être synthétisés par tout procédé connu, en particulier par voie radicalaire, par catalyse Ziegler-Natta ou par catalyse métallocène.

Les copolymères selon l'invention sont avantageusement solides à la température ambiante (25°C).

La copolymérisation peut être effectuée en masse, en solution ou en dispersion.

Les copolymères d'oléfines cristallins convenant dans la présente invention ont un point de fusion inférieur à 150°C, de préférence inférieur ou égal à 110°C.

Les copolymères de l'invention ont de préférence une masse molaire moyenne en poids $\overline{M}_w \geq 30\ 000$ (de préférence encore $\overline{M}_w \geq 40\ 000$) et un indice de polymolécularité $\frac{M_w}{M_n} \leq 3,5$ et de préférence $\leq 2,5$, $\overline{M}_n$ étant la masse molaire moyenne en nombre.

Le taux de cristallinité des copolymères est déterminé comme cela est bien connu par calorimétrie différentielle à balayage (DSC) ou par diffraction des rayons-X pour les faibles taux de cristallinité.

Les copolymères d'oléfines préférés selon l'invention sont les copolymères d'oléfines obtenus par catalyse métallocène.

Cette voie de synthèse permet en effet un très bon contrôle des poids moléculaires des copolymères et conduit à une faible polydispersité (Indice de polymolécularité $\leq 2$). Elle permet un très bon contrôle de l'incorporation du comonomère dans les chaînes de polymèresqui sont de composition chimique très voisine. De ce fait, on obtient un très bon contrôle de la cristallinité, c'est-à-dire du taux de cristallinité, de sa reproductibilité, et de la nature et dimension des cristallites formées.

Pour plus de détails quant aux avantages de cette synthèse par catalyse métallocène, on pourra se reporter aux articles "Emerging Technologies In Polymer Science and Engineering" (Technologies émergentes dans la Science et l'Ingénierie des Polymères) M.P. ZAMORA et al. - Plactics Engineering/May 97, pages 75 à 79 et "Classification of Homogeneous Ethylene-Octene Copolymers Based on Comonomer Content" (Classification des Copolymères Ethylène-Octène homogènes basée sur la teneur en Comonomère) S. BENSASON et al. - Journal of Polymer Science - Part B : Polymer Physics - Vol. 34, 130-1315 (1996).

Dans les copolymères d'oléfines convenant dans la présente invention, la structure cristalline varie en fonction du taux de comonomère amorphe dans le copolymère.

Ainsi, dans le cas des copolymères éthylène/octène, comme le décrit l'article de S. BENSASON mentionné, lorsque la teneur en octène croît, on passe :

- pour une teneur en octène $\leq 2,5\%$ en mole à des structures cristallines bien marquées avec présence de sphérulites, structures de type lamellaire appelées Type IV et les copolymères présentent alors des taux de cristallinité supérieurs à 50% ;
- pour des teneurs en octène de l'ordre de 3% en mole à des structures encore fortement cristallines, lamellaires, mais avec des sphérulites plus petites (structure de type III), et les copolymères ont un taux de cristallinité de 38 à 50%) ;
- puis, pour des teneurs en octène de 5 à 6% en mole à des structures moins cristallines avec très peu de sphérulites et un mélange de structures en lamelles avec des "micelles frangées" (structure de type II) et les copolymères ont un taux de cristallinité de 28 à 38% ;
- et, enfin, pour des teneurs en octène de 8 à 14% en mole à des structures encore plus faiblement cristallines ne comportant plus de sphérulites ni de lamelles, mais uniquement des "micelles frangées" (structure de type I), et les copolymères ont un taux de cristallinité de 10 à 28%.

Les copolymères recommandés pour la présente invention sont ceux ayant les structures de types I et II.

Les copolymères à structure de type IV, trop cristallins, ne conviennent pas pour la présente invention.

Le copolymère peut être choisi par l'homme du métier en fonction de ses propriétés et selon l'application ultérieure souhaitée pour la composition. Ainsi, le polymère peut être filmifiable ou non.

La composition telle que définie ci-dessus, comprend avantageusement une phase grasse liquide à température ambiante et au moins un actif choisi parmi les actifs physiologiquement acceptables comme les actifs cosmétiques ou dermatologiques.

Par "phase grasse volatile", on entend tout milieu non aqueux susceptible de s'évaporer de la peau ou des lèvres, en moins d'une heure à température ambiante et pression atmosphérique. Cette phase volatile comporte notamment des huiles ayant une pression de vapeur, à température ambiante et pression atmosphérique, non nulle, allant de $10^{-3}$ à 300mm de Hg.

Un autre objet de l'invention est l'utilisation dans ou pour la fabrication d'une composition sous forme de produit coulé et comprenant au moins une phase grasse liquide physiologiquement acceptable et au moins une cire, notamment solide à température ambiante, d'un copolymère d'oléfines cristallin ayant un taux de cristallinité au plus égal à 50%, soluble ou dispersable dans ladite phase grasse liquide, présent en une quantité efficace, pour obtenir un film ne migrant pas autour de la zone où elle est appliquée comme autour des lèvres et/ou des yeux (paupières).

Un autre objet de l'invention est l'utilisation dans ou pour la fabrication d'une composition comprenant une phase grasse liquide physiologiquement acceptable et au moins un ingrédient choisi parmi les actifs physiologiquement acceptables, les matières colorantes et leurs mélanges, d'au moins un copolymère d'oléfines cristallin ayant un taux de cristallinité au plus égal à 50%, soluble ou dispersable dans ladite phase grasse liquide, présent notamment en une quantité efficace pour obtenir un film ne migrant pas dans les plis de la peau situés autour des yeux et/ou des lèvres.

L'invention a encore pour objet un procédé cosmétique pour limiter, voire supprimer, la migration d'une composition de maquillage ou de soin cosmétique de la peau ou des lèvres, contenant un phase grasse liquide et au moins un ingrédient choisi parmi les matières colorantes et les actifs physiologiquement acceptables notamment cosmétiques, consistant à introduire dans la phase grasse liquide au moins un copolymère d'oléfines cristallin ayant un taux de cristallinité au plus égal à 50%, soluble ou dispersable dans ladite phase grasse liquide, présent en une quantité efficace.

Par quantité efficace, on entend une quantité limitant la migration de la composition au cours du temps. Cette quantité dépend de la quantité d'huile et/ou de matières colorantes présentes dans la composition. Par ailleurs, la propriété de non migration augmente avec la quantité de copolymère soluble ou dispersable dans la phase grasse liquide. En pratique, le copolymère peut représenter en matière active jusqu'à 70% (en matière active ou sèche) du poids total de la composition. Avantageusement, il représente de 0,5 à 70 % en matière active de la composition et mieux de 5 à 30 %.

Le copolymère peut être solubilisé dans la phase grasse de la composition par chauffage au dessus de son point de fusion.

Une première classe de copolymères d'oléfines cristallins, utilisables dans les compositions selon l'invention, sont les copolymères d'$\alpha$-oléfine, en particulier d'$\alpha$-oléfine en $C_2$-$C_{16}$ et mieux en $C_2$-$C_{12}$. De préférence, ces copolymères sont des bi- ou terpolymères et tout particulièrement des bipolymères.

Parmi les bipolymères recommandés pour les compositions de l'invention, on peut citer les bipolymères d'éthylène et d'$\alpha$-oléfine en $C_4$-$C_{16}$, de préférence en $C_4$-$C_{12}$ et les bipolymères de propylène et d'$\alpha$-oléfine en $C_4$-$C_{16}$, de préférence en $C_4$-$C_{12}$. De préférence encore, l'$\alpha$-oléfine est choisie parmi le butène-1, le pentène-1, l'hexène-1, l'octène-1, le nonène-1, le décène-1, l'undécène-1, le dodécène-1, le 3,5,5-triméthylhexène-1, le 3-méthylpentène-1, et le 4-méthylpentène-1.

Parmi ces monomères, le butène-1 et l'octène-1 sont particulièrement préférés.

Les bipolymères recommandés sont les élastomères ayant un taux de cristallinité allant de 10 à 35 %.

Ces bipolymères sont préférentiellement synthétisés par catalyse métallocène.

De tels bipolymères sont commercialisés par la Société DOW CHEMICAL sous les dénominations commerciales « AFFINITY » (plastomères) et « ENGAGE » (élastomères).

Des bipolymères éthylène-butène sont commercialisés par la Société EXXON sous l'appellation commerciale « EXACT RESINS » et par la société ELENAC sous l'appellation commerciale « LUFLEXEN ».

Parmi les terpolymères, on peut citer les terpolymères d'éthylène, de propylène et d'$\alpha$-oléfine en $C_4$-$C_{16}$, de préférence $C_4$-$C_{12}$.

Dans ces terpolymères, les teneurs en $\alpha$-oléfine en $C_4$-$C_{16}$ sont comme indiquées précédemment et les $\alpha$-oléfines préférées sont le butène, l'hexène et l'octène.

Une seconde classe de copolymères d'oléfines convenant pour l'invention sont les copolymères d'éthylène ou de propylène et d'une cyclooléfine, en particulier les bipolymères.

Généralement, la teneur en cyclooléfine des copolymères est inférieure à 20% en mole.

Parmi les cyclooléfines utilisables, on peut citer le cyclobutène, le cyclohexène, le cyclooctadiène, le norbornène, le diméthano-octahydronaphtalène (DMON), l'éthylidène norbornène, le vinyl norbornène et le 4-vinylcyclohexène.

Les copolymères recommandés de cette classe sont les copolymères d'éthylène et de norbornène. La teneur en norbornène de ces copolymères est généralement inférieure à 18% en mole pour présenter le caractère cristallin requis et ces copolymères sont synthétisés par catalyse métallocène.

Des copolymères éthylène/norbornène appropriés sont commercialisés par les Sociétés MITSUI PETROCHEMICAL ou MITSUI-SEKKA sous la dénomination commerciale « APPEL » et par la Société HOECHST-CELANESE sous la dénomination commerciale « TOPAS ».

D'autres copolymères d'éthylène/cyclooléfine recommandés sont les bipolymères éthylène/cyclobutène et éthylène/cyclohexène à faible teneur en cyclooléfine, généralement inférieure à 20% en mole.

Une troisième classe de copolymères d'oléfines appropriés est constituée par les copolymères d'oléfines de tacticité contrôlée, c'est-à-dire des copolymères comportant des motifs de tacticité différente.

Parmi ces copolymères de tacticité contrôlée, on peut citer les copolymères propylène isotactique/propylène atactique et propylène syndiotactique/propylène atactique.

Les motifs ou séquences iso- ou syndiotactiques confèrent au copolymère le caractère cristallin, cependant que les motifs ou séquences atactiques amorphes empêchent une trop forte cristallinité du copolymère et règlent le taux de cristallinité ainsi que la morphologie et la taille des cristallites.

La teneur en motifs iso- ou syndiotactiques, motifs conférant le caractère cristallin au copolymère, est donc déterminée pour obtenir le pourcentage de cristallinité voulu (≤50%) dans le copolymère.

La teneur en motifs tactiques est généralement comprise dans la gamme allant de 10 à 80% en mole. Toutefois, de préférence, la teneur en motifs tactiques est inférieure à 30% en mole.

Ces copolymères sont synthétisés par catalyse métallocène.

Une quatrième classe de copolymères d'oléfines convenant pour la présente invention, est constituée par les copolymères de monooléfine et de monomère à liaison(s) éthylénique(s) tel que les diènes, par exemple les bipolymères éthylène/butadiène, propylène/butadiène, éthylène/isoprène et propylène/isoprène, et les terpolymères éthylène/propylène/diène, obtenus également par synthèse métallocène.

La proportion de motifs « éthylène » ou « diène » dans le copolymère à cristallisation contrôlée est généralement comprise dans la gamme allant de 3 à 20% en mole.

Pour améliorer le réglage de la cristallinité du copolymère, on peut éventuellement ajouter à la composition selon l'invention des additifs gênant la cristallisation. Ces additifs, bien qu'utilisés en faible proportion, constituent des « sites » de germination nombreux et petits répartis uniformément dans la masse. Ces additifs sont typiquement des cristaux d'une substance organique ou minérale.

On peut également ajuster le taux de cristallisation, la taille et la morphologie des copolymères d'oléfines selon l'invention en mélangeant un premier copolymère d'oléfines selon l'invention avec un second polymère ou copolymère cristallin, compatible en partie avec le premier copolymère d'oléfines. Le second polymère ou copolymère peut être un copolymère d'oléfines selon l'invention, mais de taux de cristallinité différent de celui du premier copolymère, y compris un taux de cristallinité plus élevé que le taux de cristallinité des copolymères d'oléfines selon l'invention

Le deuxième polymère cristallisable peut aussi être un polymère de nature différente, par exemple un copolyéthylène/acétate de vinyle obtenu par copolymérisation radicalaire ou même un polyéthylène cristallisable tel que ceux habituellement utilisés dans le domaine cosmétique

Pour plus de détails quant à cette méthode d'ajustement du taux de cristallinité, on se référera aux articles intitulés "Elastomeric blends of homogeneous ethylene-octene copolymers (Mélanges élastomériques de copolymères homogènes éthylène-octène)" S. Bensason et al., Polymer, Volume 38, N° 15, 1997, pages 3913-19, et "Blends of homogeneous ethylene-octene copolymers (Mélanges de copolymères homogènes éthylène-octène)" S; Bensason et al., Polymer, Volume 38, N° 14, 1997, pages 3513-20.

La phase grasse liquide dans laquelle est dispersé le copolymère peut comprend une ou plusieurs huiles cosmétiquement ou dermatologiquement acceptable, et de façon générale physiologiquement acceptable, notamment choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

On peut ainsi citer les huiles hydrocarbonées d'origine animale comme le perhydrosqualène, l'huile de vison ou de tortue ; les hydrocarbures linéaires ou ramifiés d'origine minérale ou synthétique comme l'huile de paraffine ou de vaseline, le polyisobutylène hydrogéné (parléam), l'isododécane, le polydécène ou encore les 'ISOPARs', isoparaffines volatiles ; les huiles hydrocarbonées d'origine végétale comme les triglycérides liquides d'acides gras en $C_4$ à $C_{10}$ comme de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique; les esters et éthers gras tels que les esters et éthers dérivés d'acides ou d'alcools à longue chaîne (c'est-à-dire ayant de 6 à 30 atomes de carbone), notamment les esters de formule RCOOR', dans laquelle R représente le reste d'un acide gras supérieur comportant de 7 à 19 atomes de carbone, et R' représente une chaîne hydrocarbonée comportant de 3 à 20 atomes de carbone, tels que les palmitates, les adipates et les benzoates, notamment l'adipate de diisopropyle, le myristate d'isopropyle, le palmitate d'isopropyle, le stéarate de butyle, le laurate d'hexyle, l'isononanoate d'isononyle, le palmitate de 2-éthylhexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triméllitate de tridécyle, le triisostéarate de glycérine ou de diglycérine ; les acides gras supérieurs à au moins 12 atomes de carbone tels que l'acide myristique, l'acide palmitique, l'acide stéarique, l'acide béhénique, l'acide oléique, l'acide linoléique, l'acide linolénique ou l'acide isostéarique ; les alcools gras supérieurs à au moins 12 atomes de carbone tels que l'alcool stéarylique ou l'alcool oléique, l'alcool linoléique ou linolénique, l'alcool isostéarique ou l'octyl dodécanol ; les huiles siliconées telles que les polydiméthylsiloxane (PDMS) linéaires ou cycliques, volatiles ou non ; les PDMS phénylés tels que les phényltriméthicones, les phényltriméthylsiloxydiphényl-siloxanes, les diphénylméthyl diméthyltrisiloxanes, les diphényl-diméthicones, les phényl diméthicones, les polyméthylphénylsiloxanes ; les PDMS substitués par des groupements aliphatiques et/ou aromatiques, éventuellement fluorés, ou par des groupements fonctionnels tels que des groupements hydroxyle, thiol et/ou amine ; les polysiloxanes modifiés par des acides gras, des alcools gras ou des polyoxyalkylènes en particulier des polyoxyéthylènes ou copoly(oxyéthylène - oxypropylène)s tels que les diméthicone-copolyols, les silicones portant à la fois des groupes hydrophobes hydrocarbonés (par exemple des groupes alkyle en $C_2$-$C_{30}$) et des séquences ou greffons polyoxyéthylénés ou copoly(oxyéthylénés/ oxypropylénés) telles que les alkyl-diméthiconecopolyols ; les silicones fluorées ou perfluorées telles que les polydiméthyl siloxanes perfluoroalkylés et les polyméthylphénylsiloxanes perfluoro alkylés ; les huiles fluorées et notamment perfluorées ; leurs mélanges.

Avantageusement, on peut utiliser une ou plusieurs huiles volatiles à température ambiante. Après évaporation de ces huiles, on obtient un dépôt filmogène souple, non collant sur la peau ou les muqueuses, suivant respectivement le mouvement de la peau ou des lèvres, sur lesquelles la composition est appliquée. Ces huiles volatiles facilitent, en outre, l'application de la composition sur la peau, les muqueuses, les phanères.

Ces huiles peuvent être des huiles hydrocarbonées, des huiles silicones comportant éventuellement des groupements alkyle ou alcoxy en bout de chaîne siliconée ou pendante.

Comme huile de silicone volatile utilisable dans l'invention, on peut citer les silicones linéaires ou cycliques ayant de 2 à 7 atomes de silicium, ces silicones comportant éventuellement des groupes alkyle ou alcoxy ayant de 1 à 10 atomes de carbone ainsi que les isoparaffines en $C_8$ à $C_{16}$ et les huiles fluorées ou perfluorées volatiles. Ces huiles volatiles représentent notamment de 30 à 97,99% du poids total de la composition, et mieux de 30 à 75%.

Comme huile volatile utilisable dans l'invention, on peut citer notamment l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane ou les isoparaffines en $C_8$-$C_{16}$ telles que les 'ISOPARs', les PERMETYLs et notamment l'isododécane ou l'isohexadécane, et leurs mélanges.

Comme huile, on peut également citer les solvants, utilisés seuls ou en mélange, choisis parmi :

(i) les esters linéaires, ramifiés ou cycliques, ayant plus de 6 atomes de carbone,
(ii) les éthers ayant plus de 6 atomes de carbone,
(iii) les cétones ayant plus de 6 atomes de carbone.

Le choix du milieu non aqueux est effectué par l'homme du métier en fonction de la nature des monomères constituant le copolymère.

De plus, la phase grasse liquide dans laquelle est dissous ou dispersé le copolymère peut représenter de 5% à 97,99% du poids total de la composition, et de préférence de 30 à 75%.

La composition peut comprendre une matière colorante contenant un ou plusieurs composés pulvérulents et/ou un ou plusieurs colorants liposolubles, par exemple à raison de 0,01 à 70% du poids total de la composition. Les composés pulvérulents peuvent être choisis parmi les pigments et/ou les nacres et/ou les charges habituellement utilisés dans les compositions cosmétiques ou dermatologiques. Les composés pulvérulents peuvent représenter de 0,1 à 98% du poids total de la composition, et par exemple de 1 à 80%. De préférence ces composés pulvérulents représentent de 0,1 à 40%, et mieux de 1 à 30%.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon (Orgasol de chez Atochem), de poly-b-alamine et de polyéthylène, le Téflon, la lauroy-lysine, l'amidon, le nitrure de bore, les poudre de polymères de tétrafluoroéthylène, les microsphères creuses telles l'Expancel (Nobel Industrie), le polytrap (Dow Corning) et les microbilles de résine de silicone (Tospearls de Toshiba, par exemple), le carbonate de calcium précipité, la carbonate et l'hydrogèno-carbonate de magnésium, l'hydroxyapatite, les microsphères de silice creuses (Silica Beads de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stearate de zinc, de magnésium ou de lithium, le laurate de zinc, le myristate de magnésium.

Les charges utilisées, en particulier les charges organiques de nature polymèrique, peuvent être réticulées ou non et contenir à l'intérieur des particules un ou plusieurs actifs physiologiques et notamment cosmétiques ou dermatologiques pouvant être libérés sur la peau ou les lèvres après application de la composition.

Les pigments et les charges peuvent être ou non enrobés superficiellement, en particulier traités en surface par des silicones, des acides aminés, des dérivés fluorés ou toute autre substance favorisant la dispersion et la compatibilité du pigment dans la composition.

Les colorants liposolubles sont par exemple le rouge Soudan, le DC Red 17, le DC Green 6, le b-carotène, l'huile de soja, le brun Soudan, le DC Yellow 11, le DC Violet 2, le DC orange 5, le jaune quinoléine. Ils peuvent représenter de 0,01 à 20 % du poids de la composition et mieux de 0,1 à 6 %.

Le copolymère de la composition de l'invention permet la formation d'un film sur les matières kératiniques et notamment la peau et les lèvres, formant un réseau piégeant les matières colorantes et/ou les actifs. Selon la quantité relative de matières colorantes, utilisées par rapport à la quantité de polymère stabilisé, utilisée, il est possible d'obtenir un film plus ou moins brillant et plus ou moins migrant.

Comme actifs utilisables dans la composition de l'invention, on peut citer les hydratants, vitamines, acides gras essentiels, sphingolipides, filtres solaires, les antioxydants, les agents anti-acné, appaisants, bronzants (en l'absence de rayonnement UV), les agents dépigmentants, mâtifiants et leurs mélanges. Ces actifs sont utilisés en quantité habituelle pour l'homme et notamment à des concentrations de 0,001 à 20 % du poids total de la composition.

La composition selon l'invention peut, de plus, comprendre, selon le type d'application envisagée, les constituants classiquement utilisés dans les domaines considérés, qui sont présents en une quantité appropriée à la forme galénique souhaitée.

En particulier, elle peut comprendre, outre, la phase grasse liquide dans laquelle le polymère est solubilisé ou dispersé, des phases grasses additionnelles qui peuvent être choisies parmi les cires, les huiles, les gommes et/ou les corps gras pâteux, d'origine végétale, animale, minérale ou de synthèse, voire siliconés, et leurs mélanges.

Parmi les cires solides à température ambiante, susceptibles d'être présentes dans la composition de l'invention, on peut citer les cires ayant un point de fusion supérieur à 45 °C comme les cires hydrocarbonées telles que la cire d'abeilles, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch, les huiles hydrogénées (jojoba, ricin), les esters d'acides gras comme le stéarate d'octa-cosanyle. On peut également utiliser des cires de silicone, parmi lesquelles on peut citer les alkyl, alcoxy et/ou esters de polyméthylsiloxane, les chaînes alkyle, alcoxy et éther ayant de 2 à 40 atomes de carbone.

Parmi les corps gras pâteux, on peut citer la lanoline et ses dérivés (acétylés notamment), les PDMS ayant une chaîne alkyle en $C_8$ à $C_{24}$, les esters de silicone. Ces corps gras ont une température de fusion allant de 25 à 45°C et représente notamment de 0 à 50 % du poids de la composition.

Les cires peuvent être présentes à raison de 0-50 % en poids dans la composition et mieux de 10 à 30 %.

La composition peut comprendre, en outre, tout additif usuellement utilisé dans de telles compositions, tel que des épaississants, des parfums, des conservateurs, des tensioactifs. Bien entendu l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

Parmi les épaississants, on peut citer les bentonites, les silices traitées, les gommes de guar alkylées liposolubles, les polymères séquencés ou greffés comportant au moins une séquence soluble dans la composition et une séquence insoluble tel que, par exemple, les copolymères bi ou triséquencés polystyrène/copoly(éthylène-propylène) ou polystyrène/copoly(éthylène-butylène), les (polyvinyl)pyrrolidone/hexadiène, les gommes de silicone et les silicones du type KSG.

Les gommes de silicone ont en général une masse molaire moyenne en nombre comprise entre 200.000 et 1.000.000. A titre d'exemple de gommes de silicone qui peuvent être utilisées seules ou sous forme de mélange dans un solvant, on peut citer les copolymères suivants :

- poly[(diméthylsiloxane)/(méthylvinylsiloxane)]
- poly[(diméthylsiloxane)/(diphénylsiloxane)]
- poly[(diméthylsiloxane)/(phénylméthylsiloxane)]
- poly[(diméthylsiloxane)/(diphénylsiloxane)/(méthylvinylsiloxane)] ;

et les mélanges suivants :

- les mélanges formés à partir d'un polydiméthylsiloxane hydroxylé en bout de chaîne et d'un polydiméthylsiloxane cyclique ;
- les mélanges formés à partir d'une gomme de polydiméthyl siloxane et d'une silicone cyclique ; et
- les mélanges de deux polydiméthylsiloxanes de viscosités différentes.

Les compositions selon l'invention peuvent en outre contenir des homopolymères et copolymères liposolubles et/ou dispersables dans la phase grasse, différents des copolymères d'oléfines à cristallisation contrôlée selon l'invention.

Parmi ces homopolymères et copolymères, on peut citer des polyoléfines ou polyalkylènes telles que le polyéthylène, le polybutène et le polydécène ; des copolymères d'esters et/ou amides (méth)acryliques ; des copolymères d'esters vinyliques, par exemple des copolymères éthylène/acétate de vinyle ; des homopolymères ou copolymères vinyliques ou (méth)acryliques portant éventuellement un groupement silicone tels que par exemple des copolymères greffés à squelette (méth)acrylique et greffons de silicone macromère ; des copolymères à squelette ou séquences (méth)acrylique et à greffons ou séquences hydrocarbonés, par exemple polyisobutylène ; des copolymères greffés ou séquencés à squelette ou séquence polyorgano-siloxane et à greffons ou séquences (méth)acryliques et/ou vinyliques ; des homopolymères ou copolymères fluorés ou perfluorés, par exemple des polyéthers perfluorés tels ceux commercialisés sous la désignation « Fomblins », des homo ou copolymères (méth)acryliques perfluorés, des homo ou copolymères vinyliques fluorés, des homo ou copolymères d'oléfines fluorés et des poly(éther vinyliques) fluorés, les dérivés ou copolymères de polyvinylpyrrolidone.

Les compositions auxquelles s'applique l'invention peuvent être préparées de manière usuelle par l'homme du métier. Elle peuvent se présenter sous forme d'un produit coulé et par exemple sous la forme d'un stick ou bâton, ou sous la forme de coupelle utilisable par contact direct ou l'éponge. En particulier, elles trouvent une application en tant que fond de teint coulé, fard à joues ou à paupières coulé, rouge à lèvres, base ou baume de soin pour les lèvres, produit anti-cernes. Elles peuvent aussi se présenter sous forme d'une pâte souple ou encore de gel, de crème plus ou moins fluide. Elles peuvent alors constituer des fonds de teint ou des rouges à lèvres, des produits solaires ou de coloration de la peau, des eye-liners, des produits de maquillage du corps. A titre indicatif, une crème plus ou moins fluide peut être caractérisée par une viscosité newtonienne inférieure à 10Pa.s, une pate souple par une viscosité newtonienne inférieure à 50 Pa.s et un gel est en général plus épais et peut être caractérisé par un angle de déphasage inférieure à 45° sur le plateau viscoélastique linéaire. Ces mesures sont effectuée sur un rhéomètre Haake RS75 avec le mobile le plus adapté à la texture étudiée.

Les compositions de l'invention sont avantageusement anhydres et peuvent contenir moins de 5% d'eau par rapport au poids total de la composition. Elles peuvent alors se présenter notamment sous forme de gel huileux, de liquide huileux ou huile, de pâte ou de stick. Ces formes galéniques sont préparées selon les méthodes usuelles des domaines considérés.

Les compositions selon l'invention peuvent également avantageusement se présenter sous forme d'émulsion eau-dans-huile, huile-dans-eau ou eau-dans-cire, dans lesquelles les copolymères d'oléfines selon l'invention sont utilisés pour remplacer tout ou partie des cires habituellement présentes dans ces émulsions. En particulier, la cire peut comprend au moins un copolymère d'oléfines selon l'invention et au moins une huile, volatile ou non. La composition de l'invention peut aussi se présenter sous forme de dispersion vésiculaire contenant des lipides ioniques et/ou non ioniques.

Ces compositions peuvent constituer notamment une composition cosmétique de protection, de traitement ou de soin pour le visage, le cou, ou le corps, une composition de maquillage ou une composition de bronzage artificiel.

Les exemples suivants, non limitatifs, illustrent la présente invention.

**Exemple 1** : rouge à lèvres non migrant.

- Octyl dodécanol     27 g
- Tridécyl trimellitate     11,5 g
- Lanoline acétylée (patêux)     17,5 g
- Poly-isobutène hydrogéné     15 g
- Exact 4049     1 g
- Polyéthylène     7 g
- Stéarate d'octa-cosanyle     4 %
- Copolymère de PVP/eicosène     2 %
- Pigments     15 %

**Exemple 2** : rouge à lèvres non migrant.

- Engage 8400     18 %

- Pigments (oxydes de fer)    6 %
- Isododécane    50 %
- Huile de parléam    26 %

L'évaporation de l'huile volatile permet l'obtention d'un film dont les propriétés visco-élastiques sont modelables par la fraction d'huile plastifiante restante.

**Exemple 3** :rouge à lèvres non migrant.

- Engage 8400    9 %
- Pigments (oxydes de fer)    8 %
- Huile de parléam    83 %

Cette composition de l'exemple 3 a été comparée à des compositions simples selon l'art antérieur contenant respectivement du $SiCl_4$ (contre exemple 1), de la cire de polyéthylène de poids moléculaire moyen de 500 (contre exemple 2). Le contre exemple 2 est considéré par les personnes du métier (formulateurs en cosmétique) comme non migrant.

La composition 3 a aussi été comparée à un rouge à lèvres en stick disponible sur le marché : « Rouge Virtuale » commercialisé par L'ORÉAL, contenant 8 % de pigments, 64,2 % d'huiles hydrocarbonées et esters, 16,1 % d'huile de silicone, 10,5 % d'un mélange de cires, 0,9 % de charges, une quantité efficace de conservateurs et 0,2 % d'actifs cosmétiques. Cette composition notée contre exemple 3, est considérée comme migrante par les personnes du métier.

La migration est mesurée sur du papier Whathman N° 1 et à l'aide d'une petite spatule, on effectue 10 stries sur une plage de 15x15 mm2 perpendiculairement à la plus grande dimension. 3,75 mg (soit 5mg/cm2) de composition à tester sont appliqués au pinceau sur une plage de 15 x 5 mm2. La face arrière du support papier est alors imbibée de 2 gouttes de sébum artificiel, conservé au congélateur, contenant : 45 % de trioléïne, 19 % de squalène, 5 % de cholestérol et 31 % d'acide oléïque.

Le support est alors enroulé sur lui même afin de simuler le mouvement des lèvres pendant 4 min. Les stries s'ouvrent et se referment induisant alors un phénomène de capillarité : la phase coloré peut lors migrer. La présence de sébum artificiel est nécessaire ainsi que les mouvements pour provoquer cette migration.

A partir de photographies avec un Polaroïd au grossissement 3 des supports papier, on repère les contours de la phase colorée avant et après les mouvements imposés au support. La migration est alors définie comme le rapport entre la surface de migration et la surface avant migration, soit :

$$Migration = \frac{surface\ après\ mouvement - surface\ avant\ mouvement}{surface\ avant\ mouvement}$$

Les résultats présentés sont la moyenne de 10 essais, l'incertitude sur les valeurs données est de 10 %.

| | |
|---|---|
| Exemple 3 | 0,2 |
| Contre exemple 1 | 0,9 |
| Contre exemple 2 | 0,8 |
| Contre exemple 3 | 0,4 |

La phase colorée de la composition 2 selon l'invention migre 3 fois moins que celle des contre exemples 1 et 2, et 2 fois moins que celle d'un rouge à lèvres du commerce. On peut donc en déduire que les propriétés de non migrations seront encore plus exacerbées dans un rouge à lèvres en stick que dans un rouge à lèvres en crème ou pâte.

Avec les mêmes compositions, les inventeurs on étudié la migration de la phase grasse. Ils ont pour cela, appliqué sur un disque de 15 mm de diamètre formé sur un support de papier mouchoir, 8,8 mg de composition à tester. L'application est voisine de 5mg/cm2. Ils ont effectué 10 dépôts de composition pour chaque échantillon. Les supports ont été gardés pendant 12 heures dans une étuve à 34 °C.

Par transparence, on a mesuré le diamètre du nouveau disque. La migration est lors définie comme le rapport :

$$\frac{(\text{diamètre du disque après migration})^2 - (\text{diamètre du disque avant migration})^2}{(\text{diamètre du disque avant migration})^2}$$

Les résultats sont donnés avec une incertitude de 10 %.

| Exemple 3 | 2 |
| Contre exemple 1 | 3,5 |
| Contre exemple 2 | 3 |
| Contre exemple 3 | 3,3 |

La migration de la phase grasse est moins importante pour la composition 2 selon l'invention que pour les autres compositions gélifiées.

## Revendications

1.  Utilisation dans une composition pour matières kératiniques, comprenant une phase grasse liquide d'une quantité efficace d'au moins un copolymère soluble ou dispersable dans la phase grasse liquide, choisi parmi les copolymères d'oléfines cristallins ayant un taux de cristallinité au plus égal à 50 %, de préférence allant de 5 à 40%, et mieux allant de 10 à 35%, pour limiter et/ou supprimer la migration de ladite composition.

2.  Utilisation dans une composition non migrante pour matières kératiniques, comprenant une phase grasse liquide d'une quantité efficace d'au moins un copolymère soluble ou dispersable dans la phase grasse liquide, choisi parmi les copolymères d'oléfines cristallins ayant un taux de cristallinité au plus égal à 50 %, de préférence allant de 5 à 40%, et mieux allant de 10 à 35%.

3.  Utilisation selon la revendication 1 ou 2, caractérisée en ce que le copolymère représente au moins 2% en poids, par rapport au poids total de la composition.

4.  Utilisation selon l'une des revendications précédentes, caractérisée en ce que le copolymère est solide.

5.  Utilisation selon l'une des revendications précédentes caractérisée en ce que le copolymère présente une masse moléculaire moyenne en poids $\overline{M}_w$ telle que $\overline{M}_w \geq 30\,000$ et de préférence $\geq 40\,000$.

6.  Utilisation selon l'une des revendications précédentes caractérisée en ce que le copolymère a un indice de polymolécularité de $\overline{M}_w/\overline{M}_n < 3,5$, de préférence $\leq 2,5$ avec $\overline{M}_n$ étant la masse molaire moyenne en nombre.

7.  Utilisation selon l'une des revendications précédentes, caractérisée par le fait qu'elle se présente sous la forme d'un produit coulé comprenant une phase grasse liquide physiologiquement acceptable et au moins une cire solide à température ambiante.

8.  Utilisation selon l'une des revendications précédentes, caractérisée par le fait que le copolymère est filmifiable.

9.  Utilisation selon la revendication 7, caractérisée par le fait que la phase grasse liquide comprend au moins une huile volatile à température ambiante.

10.  Utilisation selon l'une des revendications précédentes, caractérisée par le fait que la composition comprend en outre, au moins un actif choisi parmi les actifs physiologiquement acceptables.

11.  Utilisation selon l'une quelconque des revendications précédentes, caractérisée par le fait qu'elle comprend, de plus, au moins une matière colorante.

12.  Utilisation selon l'une quelconque des revendications précédentes, caractérisée par le fait que le copolymère d'oléfines est choisi parmi :

   (A) les copolymères d'α-oléfines, les copolymères d'oléfines et de cyclooléfines, les copolymères d' α-oléfines et de monomère à liaison(s) éthylénique(s); et

(B) les copolymères d'α-oléfines à motifs tactiques et atactiques.

**13.** Utilisation selon la revendication 12, caractérisée par le fait que les copolymères d'α-oléfines sont choisis parmi les bipolymères d'éthylène ou de propylène et d'α-oléfine en $C_4$-$C_{16}$, de préférence $C_4$-$C_{12}$, et les terpolymères d'éthylène, de propylène et d'α-oléfine en $C_4$-$C_{16}$, de préférence $C_4$-$C_{12}$.

**14.** Utilisation selon la revendication 13, caractérisée par le fait que l'a-oléfine en $C_4$-$C_{16}$ est choisie parmi le butène-1, le pentène-1, l'hexène-1, l'heptène-1, l'octène-1, le nonène-1, le décène-1, l'undécène-1, le dodécène-1, le 3,3,5-triméthylhexène-1, le 3-méthyl pentène-1 et le 4-méthylpentène-1.

**15.** Utilisation selon la revendication 13 ou 14, caractérisée par le fait que le pourcentage en mole d'α-oléfine est au plus égal à 40%, de préférence égal ou inférieur à 30%.

**16.** Utilisation selon la revendication 12, caractérisée par le fait que les copolymères d'oléfines sont choisis parmi les bipolymères d'éthylène ou de propylène avec le cyclobutène, le cyclohexène, le cyclo octadiène, le norbornène, le diméthano-octahydronaphtalène (DMON), l'éthylidènenorbornène, le vinylnorbornène et le 4-vinylcyclohexène et les terpolymères d'éthylène, de propylène et des cyclooléfines précédentes.

**17.** Utilisation selon la revendication 16, caractérisée par le fait que le copolymère d'oléfine et de cyclooléfine contient moins de 20% en mole de cyclooléfine.

**18.** Utilisation selon la revendication 17, caractérisée par le fait que le copolymère d'α-oléfine et de cyclooléfine est un copolymère éthylène/norbornène contenant moins de 18% en mole de norbornène.

**19.** Utilisation selon la revendication 12, caractérisée par le fait que les copolymères d'α-oléfine et de monomère à liaison(s) éthylénique(s) sont choisi parmi les bipolymères éthylène/butadiène et éthylène/isoprène.

**20.** Utilisation selon la revendication 19, caractérisée par le fait que le bipolymère contient moins de 20% en mole de monomères à liaison(s) éhylénique(s).

**21.** Utilisation selon la revendication 12, caractérisée par le fait que les copolymères d'α-oléfines à motifs tactiques et atactiques sont choisis parmi les polypropylènes à motifs isotactiques et atactiques et les polypropylènes à motifs syndiotactiques et atactiques.

**22.** Utilisation selon la revendication 21, caractérisée par le fait que le taux de motifs tactiques est inférieur à 30% en mole.

**23.** Utilisation selon l'une des revendications précédentes, caractérisée par le fait que le copolymère d'oléfines est obtenu par synthèse métallocène.

**24.** Utilisation selon l'une des revendications précédentes, dans laquelle la phase grasse liquide comprend au moins une huile choisie parmi les huiles hydrocarbonées d'origine animale ou végétale ;les hydrocarbures d'origine minérale ou de synthèse ; les esters d'acide lanolique, d'acide oléique, d'acide laurique, d'acide stéarique ; les esters et éthers d'acides gras ; les alcools gras supérieurs ayant de 12 à 28 atomes de carbone ; les huiles siliconées éventuellement substituées ; les huiles fluorées ; et leurs mélanges.

**25.** Utilisation selon l'une des revendications précédentes, dans laquelle la composition comprend, en outre, au moins une phase grasse additionnelle choisie parmi les cires, les gommes et/ou les corps gras pâteux, d'origine végétale, animale, minérale, de synthèse, ou siliconés, et leurs mélanges.

**26.** Utilisation selon l'une quelconque des revendications 11 à 25, caractérisée en ce que la matière colorante comprend au moins un composé pulvérulent choisi parmi les charges, les pigments, les nacres et leurs mélanges.

**27.** Utilisation selon la revendication 26, caractérisée en ce que le composé pulvérulent représente 0,1 à 98% du poids total de la composition.

**28.** Utilisation selon la revendication 26 ou 27, caractérisée en ce que le composé pulvérulent représente de 1 à 30% du poids total de la composition.

29. Utilisation selon l'une des revendications précédentes, caractérisée en ce que le copolymère d'oléfines représente (en matière sèche) jusqu'à 70% du poids total de la composition.

30. Utilisation selon l'une des revendications précédentes, caractérisée en ce que la phase grasse liquide contient au moins une huile choisie parmi les isoparaffines en $C_8$-$C_{16}$ et les silicones linéaires ou cycliques ayant de 2 à 7 atomes de silicium, ces silicones comportant éventuellement des groupes alkyles ayant de 1 à 10 atomes de carbone, et leurs mélanges.

31. Utilisation selon l'une des revendications précédentes, dans laquelle la composition se présente sous forme coulée en stick ou en coupelle ; sous forme d'une pâte souple ; sous forme de gel huileux ; de liquide huileux, d'une émulsion eau-dans-huile, huile-dans-eau ou eau-dans-cire, ladite cire pouvant être constituée par un mélange d'au moins un copolymère d'oléfines cristallin ; de dispersion vésiculaire.

32. Utilisation selon l'une quelconque des revendications précédentes, dans laquelle la composition se présente sous forme anhydre.

33. Utilisation selon l'une des revendications précédentes, dans laquelle la composition se présente sous la forme d'un produit de soin et/ou de maquillage de la peau et/ou des lèvres.

34. Utilisation selon l'une des revendications précédentes, dans laquelle la composition se présente sous la forme d'un fond de teint coulé, d'un fard à joues ou à paupières coulé, d'un rouge à lèvres, d'une base ou baume de soin pour les lèvres, d'un eye-liner, d'un produit anti-cernes, ou d'un maquillage du corps.

Office européen
des brevets

**RAPPORT DE RECHERCHE EUROPEENNE**

Numéro de la demande

EP 00 40 0198

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 0 665 008 A (L'OREAL) 2 août 1995 (1995-08-02) --- | 1 | A61K7/06 |
| A | US 5 695 772 A (V. KANGA ET AL.) 9 décembre 1997 (1997-12-09) --- | 1 | |
| A | EP 0 270 339 A (MITSUI PETROCHEMICAL IND., LTD) 8 juin 1988 (1988-06-08) ----- | 1 | |

**DOMAINES TECHNIQUES RECHERCHES (Int.Cl.7)**

A61K

Le présent rapport a été établi pour toutes les revendications

| Lieu de la recherche | Date d'achèvement de la recherche | Examinateur |
|---|---|---|
| LA HAYE | 11 avril 2000 | Glikman, J-F |

CATEGORIE DES DOCUMENTS CITES

X : particulièrement pertinent à lui seul
Y : particulièrement pertinent en combinaison avec un autre document de la même catégorie
A : arrière–plan technologique
O : divulgation non–écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**EP 1 034 776 A1**

**ANNEXE AU RAPPORT DE RECHERCHE EUROPEENNE**
**RELATIF A LA DEMANDE DE BREVET EUROPEEN NO.**  EP 00 40 0198

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-04-2000

| Document brevet cité au rapport de recherche | Date de publication | Membre(s) de la famille de brevet(s) | Date de publication |
|---|---|---|---|
| EP 665008 A | 02-08-1995 | FR 2715294 A | 28-07-1995 |
| | | AT 154236 T | 15-06-1997 |
| | | BR 9500319 A | 17-10-1995 |
| | | CA 2141124 A | 27-07-1995 |
| | | CN 1111533 A | 15-11-1995 |
| | | DE 69500335 D | 17-07-1997 |
| | | DE 69500335 T | 20-11-1997 |
| | | ES 2104460 T | 01-10-1997 |
| | | HU 71730 A,B | 29-01-1996 |
| | | JP 2960658 B | 12-10-1999 |
| | | JP 7252130 A | 03-10-1995 |
| | | PL 306947 A | 07-08-1995 |
| | | US 5556613 A | 17-09-1996 |
| | | US 5750095 A | 12-05-1998 |
| US 5695772 A | 09-12-1997 | AU 713173 B | 25-11-1999 |
| | | AU 4012897 A | 19-03-1998 |
| | | BR 9706667 A | 20-07-1999 |
| | | CN 1218393 A | 02-06-1999 |
| | | CZ 9801281 A | 16-09-1998 |
| | | WO 9808488 A | 05-03-1998 |
| | | EP 0929290 A | 21-07-1999 |
| | | PL 328861 A | 01-03-1999 |
| EP 270339 A | 08-06-1988 | JP 1984410 C | 25-10-1995 |
| | | JP 7002617 B | 18-01-1995 |
| | | JP 63139103 A | 10-06-1988 |
| | | CA 1326824 A | 08-02-1994 |
| | | CN 1017964 B | 26-08-1992 |
| | | DE 3785158 A | 06-05-1993 |
| | | US 4960875 A | 02-10-1990 |

EPO FORM P0460